# NOUVEAU FASCICULE DE BREVET EUROPEEN

(11) **EP 0 861 065 B2**
(45) Date de publication et mention de la décision concernant l'opposition: **08.11.2006**
(45) Mention de la délivrance du brevet: 12.09.2001
(21) Numéro de dépôt: 97932885.3
(22) Date de dépôt: 10.07.1997
(51) Int. Cl.: A61Q 5/10

(54) **COMPOSITION DE TEINTURE D'OXYDATION POUR FIBRES KERATINIQUES COMPRENANT UN POLYMERE AMPHIPHILE NON-IONIQUE**
OXIDATIONSFÄRBEMITTEL FÜR KERATINFASERN DAS EIN NICHTIONISCHES AMPHIPHILES POLYMER ENTHÄLT
KERATIN FIBRE OXIDATION DYEING COMPOSITION CONTAINING A NON-IONIC AMPHIPHILIC POLYMER

(30) Priorité: 23.07.1996 FR 9609253
(43) Date de publication de la demande: 02.09.1998
(73) Titulaire: L'ORÉAL, 75008 Paris (FR)
(72) Inventeur: DE LA METTRIE, Roland, F-78110 Le Vésinet (FR); BOUDY, Françoise, F-75012 Paris (FR)
(74) Mandataire: Wattremez, Catherine
(86) Numéro de dépôt international: PCT/FR1997/001262
(87) Numéro de publication internationale: WO 1998/003150

(56) Documents cités:
- EP-A- 0 241 707
- EP-A- 0 412 706
- EP-A- 0 555 155
- WO-A-91/11985
- WO-A-97/24105
- WO-A-97/24107
- US-A- 4 776 855
- US-A- 5 100 658
- Rohm and Haas Bulletin "Rheology Modifiers for Personal Care Applications", Mars 1994

## Description

La présente invention concerne une composition de teinture d'oxydation des fibres kératiniques et en particulier des fibres kératiniques humaines telles que les cheveux, comprenant au moins un précurseur de colorant d'oxydation et éventuellement un ou plusieurs coupleurs et au moins un polymère amphiphile non-ionique particulier comportant au moins une chaîne grasse et au moins un motif hydrophile.

Il est connu de teindre les fibres kératiniques et en particulier les cheveux humains, avec des compositions de teinture contenant des précurseurs de colorants d'oxydation, généralement appelés "bases d'oxydation", en particulier des ortho- ou paraphénylènediamines, des ortho- ou para- aminophénols, et des bases hétérocycliques.

Les précurseurs de colorants d'oxydation sont des composés initialement peu ou pas colorés qui développent leur pouvoir tinctorial au sein du cheveu en présence d'agents oxydants en conduisant à la formation de composés colorés. La formation de ces composés colorés résulte, soit d'une condensation oxydative des «bases d'oxydation» sur elles-mêmes, soit d'une condensation oxydative des «bases d'oxydation» sur des composés modificateurs de coloration, ou «coupleurs», qui sont généralement présents dans les compositions tinctoriales utilisées en teinture d'oxydation et sont représentés plus particulièrement par des métaphénylènediamines, des méta-aminophénols et des métadiphénols, et certains composés hétérocycliques.

La variété des molécules mises en jeu, qui sont constituées d'une part par les «bases d'oxydation» et d'autre part par les «coupleurs», permet l'obtention d'une palette très riche en coloris.

Pour localiser le produit de coloration d'oxydation à l'application sur les cheveux afin qu'il ne coule pas sur le visage ou en dehors des zones que l'on se propose de teindre, on a jusqu'ici eu recours à l'emploi d'épaississants traditionnels tels que l'acide polyacrylique réticulé, les hydroxyéthylcelluloses, les cires ou encore à des mélanges d'agents tensio-actifs non-ioniques de HLB (Hydrophilic Lipophilic Balance), qui, convenablement choisis, engendrent l'effet gélifiant quand on les dilue au moyen d'eau et/ou d'agents tensio-actifs.

Le document EP-A-0 241 707 décrit une composition binaire de teinture pour les cheveux, la première étant aqueuse et alcaline, la seconde contenant de 0.04 à 25% en poids d'un polymère d'un ester d'acide carboxylique en C₁₆-C₂₂ et d'un ether de polyalkylene glycol d'un alcool en C₁₆-C₂₂ et 2 (ou plus) monomères d'un acide carboxylique en C₁₆-C₂₂ ou leurs esters.

Cependant, la demanderesse a constaté que les ingrédients du type épaississants traditionnels, tensio-actifs et solvants, freinent généralement la montée du colorant sur les fibres, ce qui se traduit par une nuance terne et aussi par une utilisation plus importante de colorant, de solvant et/ou d'agents tensio-actifs pour solubiliser le colorant, si l'on veut néanmoins obtenir une nuance puissante.
Par ailleurs, elle a également constaté qu'après mélange avec l'oxydant, les compositions tinctoriales contenant le ou les précurseurs de colorants d'oxydation et éventuellement le ou les coupleurs, et en outre lesdits ingrédients, perdaient une partie de leur caractère gélifié et engendraient par voie de conséquence des coulées indésirables.

Or, après d'importantes recherches menées sur la question, la Demanderesse vient maintenant de découvrir qu'il est possible d'obtenir des compositions de teinture d'oxydation (après mélange avec l'oxydant) qui ne coulent pas et restent donc mieux localisées au point d'application, et qui permettent aussi d'obtenir des nuances plus puissantes ou plus chromatiques (plus lumineuses), si on introduit (i) soit dans la composition contenant le ou les précurseurs de colorants d'oxydation et éventuellement le ou les coupleurs [ou composition (A)], soit (ii) dans la composition oxydante [ou composition (B)], ou (iii) dans les deux compositions à la fois, une quantité efficace d'un polymère amphiphile non-ionique comportant au moins une chaîne grasse et au moins un motif hydrophile.

Au sens de la présente invention, la chromaticité (luminosité) est définie par la valeur c* dans le système de notation colorimétrique L*, a*, b*, de la Commission Internationale de l'Eclairage (C.I.E.). Cette valeur est égale à la racine carrée de la somme a² + b² (+a est rouge, -a est vert, +b est jaune, -b est bleu). La nuance est d'autant plus lumineuse que la valeur de c* est grande.

Dans ce système de notation, L* définit la puissance de la nuance. La nuance est d'autant plus puissante que la valeur de L* est faible (0=noir, 100=blanc).

Ces découvertes sont à la base de la présente invention.

La présente invention a ainsi pour objet une composition de teinture d'oxydation pour fibres kératiniques, selon la revendication 1.

Grâce à la présente invention, il est en outre possible, et ceci de manière avantageuse, de réduire la consommation en agents tensio-actifs, voire de les supprimer.
L'invention permet également de diminuer la quantité de matières actives colorantes utilisées dans les compositions de teinture, par rapport aux techniques classiques et connues de l'art antérieur.

Un autre objet de la présente invention porte sur une composition prête à l'emploi pour la teinture des fibres kératiniques, qui contient au moins un précurseur de colorant d'oxydation et éventuellement au moins un coupleur, et au moins un polymère amphiphile non-ionique comportant au moins une chaîne grasse et au moins un motif hydrophile tel que défini ci-avant, et un agent oxydant.

L'invention vise également un procédé de teinture des fibres kératiniques, et en particulier des fibres kératiniques humaines telles que les cheveux, consistant à appliquer sur ces fibres au moins une composition (A1) contenant, dans un milieu approprié pour la teinture, au moins un précurseur de colorant d'oxydation et éventuellement au moins un coupleur, en association avec au moins un polymère amphiphile non-ionique comportant au moins une chaîne grasse et au moins un motif hydrophile tel que défini ci-avant, la couleur étant révélée à pH alcalin, neutre ou acide à l'aide d'un agent oxydant qui est mélangé juste au moment de l'emploi à la composition (A1) ou qui est présent dans une composition (B1) appliquée séquentiellement sans rinçage intermédiaire.

L'invention vise aussi une variante de ce procédé, qui consiste à appliquer sur les fibres au moins une composition (A2) contenant dans un milieu approprié pour la teinture, au moins un précurseur de colorant d'oxydation et éventuellement au moins un coupleur, et ceci en la présence ou l'absence de polymère amphiphile non-ionique comportent au moins une chaîne grasse et au moins un motif hydrophile tel que défini ci-avant, la couleur étant révélée à pH alcalin, neutre ou acide à l'aide d'une composition oxydante (B2) qui contient un agent oxydant et une quantité effficace d'au moins un polymère amphiphile non-ionique comportant au moins une chaîne grasse et au moins un motif hydrophile tel que défini ci-avant, et qui est mélangée juste au moment de l'emploi à la composition (A2) ou qui est appliquée séquentiellement sans rinçage intermédiaire.

L'invention a également pour objet des dispositifs de teinture ou « kits » à plusieurs compartiments, dont le premier compartiment contient au moins un précurseur de colorant d'oxydation, éventuellement au moins un coupleur, et au moins un polymère amphiphile non-ionique comportant au moins une chaîne grasse et au moins un motif hydrophile tel que défini ci-avant, et le deuxième compartiment un agent oxydant.
Selon une autre variante, l'invention a également pour objet des dispositifs de teinture ou « kits» à plusieurs compartiments, dont le premier compartiment contient au moins un précurseur de colorant d'oxydation, éventuellement au moins un coupleur, et ceci en la présence ou en l'absence de polymère amphiphile non-ionique comportant au moins une chaîne grasse et au moins un motif hydrophile tel que défini ci-avant, et le deuxième compartiment un agent oxydant et une quantité efficace d'au moins un polymère amphiphile non-ionique comportant au moins une chaîne grasse et au moins un motif hydrophile tel que défini ci-avant.

L'invention concerne aussi l'utilisation de la composition de teinture d'oxydation ci-dessus définie ou d'un dispositif de teinture ou « kit à plusieurs compartiments tel que défini ci-avant pour la teinture des fibres kératiniques humaines telles que les cheveux.

Mais d'autres caractéristiques, aspects, objets et avantages de l'invention apparaîtront encore plus clairement à la lecture de la description et des exemples qui suivent.

Les polymères amphiphiles non-ioniques comportant au moins une chaîne grasse et au moins un motif hydrophile, utilisés selon l'invention, sont choisis parmi :
**(1)** les celluloses modifiées par des groupements comportant au moins une chaîne grasse ; on peut citer à titre d'exemple :
   - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS (alkyles en C₁₆) vendu par la société AQUALON, ou le produit BERMOCOLL EHM 100 vendu par la société BEROL NOBEL,
   - celles modifiées par des groupes polyalkylène glycol éther d'alkyl phénol, tel que le produit AMERCELL POLYMER HM-1500 (polyéthylène glycol (15) éther de nonyl phénol) vendu par la société AMERCHOL.
**(2)** les hydroxypropylguars modifiés par des groupements comportant au moins une chaîne grasse tel que le produit ESAFLOR HM 22 (chaîne alkyle en C₂₂) vendu par la société LAMBERTI, les produits M IRACARE XC95-3 (chaîne alkyle en C₁₄) et RE205-1 (chaîne alkyle en C₂₀) vendus par la société RHONE POULENC.
**(3)** les uréthanes polyéthers comportant au moins une chaîne grasse telle que des groupes alkyle ou alcényle en C₈-C₃₀, comme les produits DAPRAL T 210 et DAPRAL T 212 vendus par la société AKZO.

Les polymères amphiphiles non-ioniques comportant au moins une chaîne grasse et au moins un motif hydrophile selon l'invention, sont utilisés de préférence en une quantité pouvant varier d'environ 0,05 à 10% en poids du poids total de la composition de teinture appliquée sur les fibres. Plus préférentiellement, cette quantité varie d'environ 0,2 à 5% en poids.

Les précurseurs de colorants d'oxydation utilisables dans le cadre de la présente invention sont choisis parmi ceux classiquement connus en teinture d'oxydation, et parmi lesquels on peut notamment citer:
- les paraphénylènediamines de formule (I) suivante et leurs sels d'addition avec un acide : 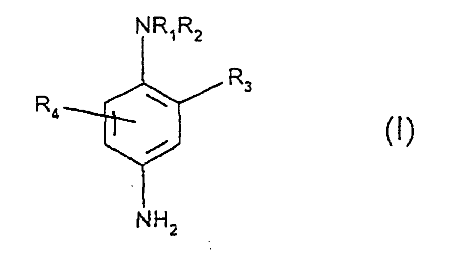
dans laquelle :
**R**_{**1**} représente un atome d'hydrogène, un radical ,alkyle en C₁-C₄, monohydroxyalkyle en C₁-C₄, polyhydroxyalkyle en C₂-C₄ ou 4'-aminophényle,
**R**_{**2**} représente un atome d'hydrogène, un radical alkyle en C₁-C₄, monohydroxyalkyle en C₁-C₄ ou polyhydroxyalkyle en C₂-C₄,
**R**_{**3**} représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄,
**R**_{**4**} représente un atome d'hydrogène ou un radical alkyle en C₁-C₄,
Parmi les paraphénylènediamines de formule (1) ci-dessus, on peut plus particulièrement citer la paraphénylènediamine, la paratoluylènediamine, la 2-chloro-paraphénylènediamine, la 2,3-diméthyl-paraphénylènedi amine, la 2,6-diméthyl-paraphénylènediamine, la 2,6-diéthyl-paraphènylènediamine, la 2,5-diméthyl-paraphénylènediamine, la N,N-diméthyl-paraphénylènediamine, la N,N-diéthyl-paraphénylènediamine, la N,N-dipropyl-paraphénylènediamine, la 4-amino-N,N-diéthyl-3-méthyl-aniline, la N,N-bis-(β-hydroxyéthyl)-paraphénylènediamine, la 4-amino-N,N-bis-(β-hydroxyéthyl)-3-méthyl-aniline, la 4-amino-3-chloro-N,N-bis-(β-hydroxyéthyl)-aniline, la 2-β-hydroxyéthyl-paraphénylènediamine, la 2-fluoro-paraphénylènediamine, la 2-isopropyl-paraphénylènediamine, la N-(β-hydroxypropyl)-paraphénylènediamine, la 2-hydroxyméthyl-paraphénylènediamine, la N,N-diméthyl-3-méthyl-paraphénylènediamine, la N,N-(éthyl, β-hydroxyéthyl)-paraphénylènediamine, la N-(β,γ-dihydroxypropyl)paraphénylènediamine, la N-(4'-aminophényl)-paraphénylènediamine, la N-phényl-paraphénylènediamine, la 2-β-hydroxyéthyloxy-paraphénylènediamine, et leurs sels d'addition avec un acide.
Parmi les paraphénylènediamines de formule (I) ci-dessus, on préfère tout particulièrement la paraphénylènediamine, la paratoluylènediamine, la 2-isopropyl-paraphénylènediamine, la 2-β-hydroxyéthyl-paraphénylènediamine, la 2-β-hydroxyéthyloxy-paraphénylenediamine, la 2,6-diméthyl-paraphénylène-diamine, la 2,6-diéthylparaphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.
- les bis-phénylalkylènediamines répondant à la formule (II) suivante, et leurs sels d'addition avec un acide : 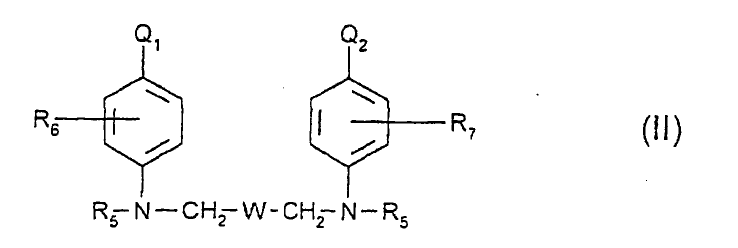
dans laquelle :
Q₁ et Q₂, identiques ou différents, représentent un radical hydroxyle ou NHR₈ dans lequel R₈ représente un atome d'hydrogène ou un radical alkyle en C₁-C₄,
**R**_{**5**} représente un atome d'hydrogène, un radical alkyle en C₁-C₄, monohydroxyalkyle en C₁-C₄, polyhydroxyalkyle en C₂-C₄ ou aminoalkyle en C₁-C₄ dont le reste amino peut être substitué,
**R**_{**6**} et **R**_{**7**}, identiques ou différents, représentent un atome d'hydrogène ou d'halogène ou un radical alkyle en C₁-C₄,
**W** représente un radical pris dans le groupe constitué par les radicaux suivants : -(CH₂)ₙ- ; -(CH₂)ₘ-O-(CH₂)ₘ- ; -(CH₂)ₘ-CHOH-(CH₂)ₘ- et 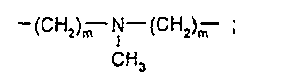 dans lesquels n est un nombre entier compris entre 0 et 8 inclusivement et m est un nombre entier compris entre 0 et 4 inclusivement.
Parmi les bis-phénylalkylènediamines de formules (II) ci-dessus, on peut plus particulièrement citer le N, N'-bis-(β-hydroxyéthyl)-N,N'-bis-(4'-aminophènyl)-1,3-diamino-2-propanol, la N,N'-bis-(β-hydroxyéthyl)-N,N'-bis-(4'-aminophényl)-éthylènediamine, la N,N'-bis-(4-aminophényl)-tétraméthylènediamine, la N,N'-bis-(β-hydroxyéthyl)-N,N'-bis-(4-aminophenyl)-tétraméthylènediamine, la N,N'-bis-(4-méthylaminophényl)-tétraméthylènediamine, la N,N'-bis-(éthyl)-N,N'-bis-(4'-amino, 3'-méthylphényl)-éthylènediamine, et leurs sels d'addition avec un acide.
Parmi ces bis-phénylalkylènediamines de formule (II), le N,N'-bis-(β-hydroxyéthyl)-N,N'-bis-(4'-aminophényl)-1,3-diamino-2-propanol ou l'un de ses sels d'addition avec un acide sont particulièrement préférés.
- les para-aminophénols répondant à la formule (III) suivante, et leurs sels d'addition avec un acide : 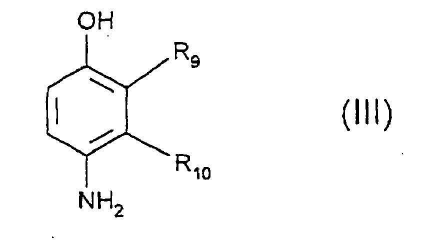
dans laquelle :
**R**_{**9**} représente un atome d'hydrogène, un radical alkyle en C₁-C₄, monohydroxyalkyle en C₁-C₄, alcoxy (C₁-C₄)alkyle(C₁-C₄) ou aminoalkyle en C₁-C₄, ou hydroxyalkyl(C₁-C₄)aminoalkyle en C₁-C₄.
**R**_{**10**} représente un atome d'hydrogène ou de fluor, un radical alkyle en C₁-C₄, monohydroxyalkyle en C₁₋C₄, polyhydroxyalkyle en C₂-C₄, aminoalkyle en C₁-C₄, cyanoalkyle en C₁-C₄ ou alcoxy(C₁-C₄)alkyle(C₁₋C₄), étant entendu qu'au moins un des radicaux R₉ ou R₁₀ représenté un atome d'hydrogène.

Parmi les para-aminophénols de formule (III) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-methyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.
- les ortho-aminophénols utilisables à titre de bases d'oxydation dans le cadre de la présente l'invention, sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide
- les bases hétérocycliques utilisables à titre de bases d'oxydation dans le cadre de la présente invention, sont notamment choisies parmi les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB-1 026 978 et GB-1 153 196, comme la 2,5-diaminopyridine, et leurs sels d'addition avec un acide.

Parmi les dérivés pyrimidiniques, on peut plus particulièrement citer les composés décrits par exemple dans les brevets allemand DE-2 359 399 ou japonais JP-88-169 571 et JP-91-333 495, comme la 2,4,5,6-tétra-aminopyrimidine, la 4-hydroxy-2,5,6-triaminopyrimidine, et leurs sels d'addition avec un acide.

Parmi les dérivés pyrazoliques, on peut plus particulièrement citer tes composés décrits dans les brevets DE-3 843 892, DE-4 133 957 et demandes de brevet WO-94/08969 et WO-94/08970 comme le 4,5-diamino-1-méthylpyrazole, le 3,4-diamino-pyrazole, le 4,5-diamino-1-(4'-chlorobenzyl)-pyrazole, et leurs sels d'addition avec un acide.

Selon l'invention, la ou les bases d'oxydation représentent de préférence, de 0,0005 à 12% en poids environ du poids total de la composition (A) et encore plus préférentiellement de 0,005 à 6% en poids environ.

Les coupleurs utilisables dans le procédé de teinture selon l'invention sont ceux classiquement utilisés dans les compositions de teinture d'oxydation, c'est-à-dire des métaphénylènediamines, des métaaminophénols et des métadiphénols, les dérivés mono- ou poly-hydroxylés du naphtalène, le sésamol et ses dérivés et des composés hétérocycliques tels que par exemple les coupleurs indoliques, les coupleurs indoliniques, les coupleurs pyridiniques et leurs sels d'addition avec un acide.

Ces coupleurs peuvent notamment être choisis parmi le 2-méthyl-5-amino-phénol, le 5-N-(β-hydroxyéthyl)-amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2,4-diamino-1-(β-hydroxyéthyloxy)-benzène, le 2-amino-4-(β-hydroxyéthylamino)-1-méthoxy-benzène, le 1,3-diamino-benzène, le 1,3-bis-(2,4-diaminophénoxy)-propane, le sésamol, l'α-naphtol, le 6-hydroxy-indole, le 4-hydroxy-indole, le 4-hydroxy-N-méthyl-indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, et leurs sels d'addition avec un acide.

Lorsqu'ils sont présents, ces coupleurs représentent de préférence de 0,0001 à 10% en poids environ du poids total de la composition (A), et encore plus préférentiellement de 0,005 à 5% en poids environ.

D'une manière générale, les sels d'addition avec un acide des bases d'oxydation et coupleurs sont notamment choisis parmi les chlorhydrates, les bromhydrates, les sulfates et les tartrates, les lactates et les acétates.

La composition (A) peut encore contenir, en plus des précurseurs de colorants d'oxydation définis ci-dessus et des éventuels coupleurs associés, des colorants directs pour enrichir les nuances en reflets. Ces colorants directs peuvent notamment alors être choisis parmi les colorants nitrés, azoïques ou anthraquinoniques.

La composition (A) et/ou la composition (B) peuvent en outre plus particulièrement contenir, au moins un polymère substantif cationique ou amphotère tel que défini aux pages 3 et 4 de la demande de brevet EP-0 673 641 A1, et dont on préfère avantageusement mettre en oeuvre les :
- les polymères de polyammonium quaternaire préparés et décrits dans le brevet français 2 270 846, constitués de motifs récurrents répondant à la formule (IV) suivante : 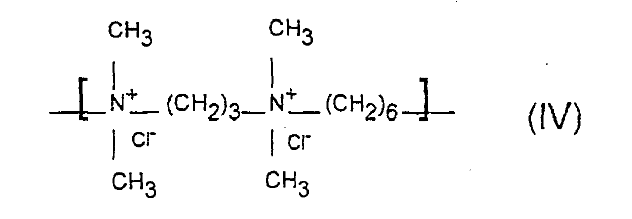 et dont le poids moléculaire, déterminé par chromatographie par perméation de gel, est compris entre 9500 et 9900 ;
- les polymères de polyammonium quaternaire préparés et décrits dans le brevet français 2 270 846, constitués de motifs récurrents répondant à la formule (V) suivante : 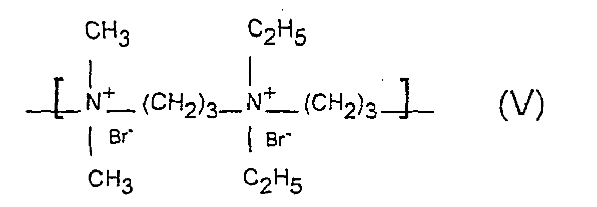 et dont le poids moléculaire, déterminé par chromatographie par perméation de gel, est d'environ 1200.

Le milieu de la composition (A) approprié pour la teinture, est de préférence un milieu aqueux constitué par de l'eau et peut éventuellement contenir des solvants organiques acceptables sur le plan cosmétique, dont plus particulièrement, des alcools tels que l'alcool éthylique, l'alcool isopropylique, l'alcool benzylique, et l'alcool phényléthylique, ou des glycols ou éthers de glycol tels que, par exemple, les éthers monométhylique, monoéthylique et mono-butylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, dans des concentrations comprises entre environ 0,5 et 20% et, de préférence, entre environ 2 et 10% en poids par rapport au poids total de la composition.

La composition (A) peut encore contenir une quantité efficace d'autres agents, par ailleurs antérieurement connus en coloration d'oxydation, tels que divers adjuvants usuels comme des séquestrants, des agents de conditionnement du cheveu, des silicones, des conservateurs, des opacifiants, etc..., et éventuellement des agents tensio-actifs anioniques, cationiques, non-ioniques, amphotères ou zwittérioniques ou leurs mélanges.

Ladite composition peut également contenir des agents antioxydants. Ceux-ci peuvent être choisis en particulier parmi le sulfite de sodium, l'acide thioglycolique, l'acide thiolactique, le bisulfite de sodium, l'acide déhydroascorblque, l'hydroquinone, la 2-méthyl-hydroquinone, la ter.butyl-hydroquinone et l'acide homogentlsique, et ils sont alors généralement présents dans des quantités allant d'environ 0,05 à 1,5% en poids par rapport au poids total de la composition.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

Dans la composition (B), l'agent oxydant est choisi de préférence parmi le peroxyde d'urée, les bromates ou ferricyanures de métaux alcalins, les persels tels que les perborates et les persulfates. L'utilisation du peroxyde d'hydrogène est particulièrement préférée.
La composition (B) est avantageusement constituée par une solution d'eau oxygénée dont le titre peut varier, plus particulièrement, d'environ 2,5 à 40 volumes, et encore plus préférentiellement d'environ 5 à 20.

Le pH de la composition prête à l'emploi et appliquée sur les fibres kératiniques [composition résultant du mélange de la composition tinctoriale (A) et de la composition oxydante (B)], est généralement compris entre les valeurs 4 et 11. II est de préférence compris entre 6 et 10, et peut être ajusté à la valeur désirée au moyen d'agents acidifiants ou alcalinisants bien connus de l'état de la technique en teinture des fibres kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (VI) suivante : 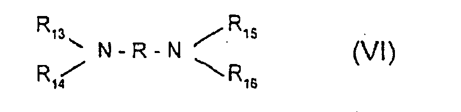 dans laquelle R est un reste propylène éventuellement substitué par un groupement hydroxyle ou un radical alkyle en C₁-C₄ ; R₁₃, R₁₄, R₁₅ et R₁₆, identiques ou différents, représentent un atome d'hydrogène, un radical alkyle en C₁-C₄ ou hydroxyalkyle en C₁-C₄.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

Le procédé de teinture selon l'invention consiste, de préférence, à appliquer un mélange, réalisé extemporanément au moment de l'emploi à partir des compositions (A) et (B) décrites ci-avant, sur les fibres kératiniques sèches ou humides, et à le laisser agir pendant un temps de pause variant, de préférence, de 1 à 60 minutes environ, et plus préférentiellement de 10 à45 minutes environ, à rincer les fibres, puis éventuellement à les laver au shampooing, puis à les rincer à nouveau, et à les sécher.

Des exemples concrets illustrant l'invention vont maintenant être donnés, sans pour autant présenter un caractère limitatif.

### EXEMPLE 1:

On a préparé la composition de teinture, conforme à l'invention, suivante

| | |
|---|---|
| NATROSOL PLUS GRADE 330 CS (Aqualon) | 1,0 g |
| Acide oléique | 3,0 g |
| Solution aqueuse de bisulfite de sodium à 35% de MA* | 0,45 g MA* |
| Paraphénylènediamine | 0,162g |
| Résorcine | 0,165g |
| Ammoniaque (20% de NH₃) | 11,5 g |
| Agent séquestrant q.s. | |
| Eau q.s.p. | 100 g |

| | |
|---|---|
| MA* = Matière Active | |

Au moment de l'emploi, on a mélangé cette composition poids pour poids avec une solution d'eau oxygénée à 20 volumes, puis on a appliqué le mélange obtenu, sur des mèches de cheveux permanentés à 90% de blancs. Après 30 minutes de pause, on a rincé les mèches, puis on les a lavées avec un shampooing, rincées à nouveau, puis on les a séchées.
A l'aide d'un spectrocolorimètre I.C.S., on a mesuré la valeur L*, dans le système international de notation de la couleur L*, a*, b*, de la C.I.E.

Le résultat a été le suivant : L* = 32,19

### EXEMPLE 2 COMPARATIF:

On a reproduit l'exemple 1, en remplaçant 1 gramme de polymère amphiphile non-ionique (NATROSOL PLUS GRADE 330 CS) par le mélange des deux tensio-actifs non-ioniques suivants (permettant d'obtenir la même viscosité) :
24 grammes d'alcool décylique (C₁₀-C₁₂-C₁₄ / 85-8,5-6,5) oxyéthyléné à 3,5 moles d'oxyde d'éthylène vendu sous la dénomination Mergital BL 309 par la société Henkel, et
16 grammes d'alcool décylique (C₁₀-C₁₂-C₁₄ / 85-8,5-6,5) oxyéthyléné à 5,5 moles d'oxyde d'éthylène vendu sous la dénomination Mergital BL 589 par la société Henkel.
On a ensuite suivi le même protocole qu'à l'exemple 1.
Le résultat a été le suivant : L* = 35,72

Conclusion : la nuance obtenue selon l'invention est plus puissante (L* plus petit) que celle obtenue selon l'art antérieur.

## Revendications

1. Composition de teinture d'oxydation pour fibres kératiniques, en particulier pour fibres kératiniques humaines telles que des cheveux, du type comprenant dans un milieu approprié pour la teinture, au moins un précurseur de colorant d'oxydation et, le cas échéant , un ou plusieurs coupleurs, **caractérisée par le fait qu**'elle contient en outre au moins un polymère amphiphile non-ionique comportant au moins une chaîne grasse et au moins un motif hydrophile choisi dans le groupe constitué par les celluloses non-ioniques modifiées par des groupements comportant au moins une chaîne grasse, les hydroxypropylguars modifiés par des groupements comportant au moins une chaîne grasse, les uréthanes polyéthers comportant au moins une chaîne grasse ; sous réserve que :
(1) ladite composition soit différente d'une composition contenant l'Aculyn 44 (nom INCI : PEG-150/Decyl Alcohol/SMDI Copolymer), un peroxyacide organique préformé oxydant et un ou plusieurs colorants d'oxydation capillaires ;
(2) ladite composition soit différente d'une composition contenant l'Aculyn 44 (nom INCI: PEG-150/Decyl Alcohol/SMDI Copolymer), un peroxygène hydrosoluble oxydant, un précurseur de peroxyacide organique oxydant et un ou plusieurs colorants d'oxydation capillaires ;
(3) ladite composition soit différente d'une composition contenant 5% de peroxyde d'hydrogène, 0,8% de para-phénylènediamine, 0,2% de para-aminophénol, 1% de méta-aminophénol, 1,6% de résorcinol, 1% de nonyoxynol-9, 0,1% d'acide ethylènediamine tétraacétique, 0,5% d'Aculyn 44 (nom INCI: PEG-150/Decyl Alcohol/SMDI Copolymer) et de l'eau.

2. Composition selon la revendications 1, **caractérisée par le fait que** les celluloses sont des hydoxyéthylcelluloses modifiées par des groupements comportant au moins un groupe alkyle, arylalkyle, alkylaryle.

3. Composition selon la revendication 2, **caractérisée par le fait que** la cellulose est une hydoxyéthylcellulose modifiée par des groupements comportant au moins un groupe alkyle en C₁₆.

4. Composition selon la les revendications 1, **caractérisée par le fait que** les celluloses sont des hydoxyéthylcelluloses modifiées par des groupements comportant au moins un groupe polyalkylène glycol éther d'alkyl phénol.

5. Composition selon la revendication 4, **caractérisée par le fait que** la cellulose est une hydoxyéthylcellulose modifiée par des groupements comportant au moins un groupe polyéthylène glycol (15) éther de nonyl phénol.

6. Composition selon la revendications 1, **caractérisée par le fait que** les uréthanes polyéthers sont modifiés par au moins un groupe alkyle ou alcényle en C₈-C₃₀.

7. Composition selon l'une quelconque des revendications précédentes, **caractérisée par le fait que** les précurseurs de colorants d'oxydation sont choisis parmi les ortho- ou para- phénylènediamines, les bis-phénylalkylènediamines, les ortho- ou para- aminophénols, et les bases hétérocycliques, ainsi que les sels d'addition de ces composés avec un acide.

8. Composition selon l'une quelconque des revendications précédentes, **caractérisée par le fait que** les précurseurs de colorants d'oxydation sont présents dans des concentrations allant de 0,0005 à 12% en poids par rapport au poids total de la composition.

9. Composition selon l'une quelconque des revendications précédentes, **caractérisée par le fait que** les coupleurs sont choisis parmi les métaphénylènediamines,les métaaminophénols, les métadiphénols, les coupleurs hétérocycliques, et les sels d'addition de ces composés avec un acide.

10. Composition selon l'une quelconque des revendications précédentes, **caractérisée par le fait que** les coupleurs sont présents dans des concentrations allant de 0,0001 à 10% en poids par rapport au poids total de la composition.

11. Composition selon l'une quelconque des revendications précédentes, **caractérisée par le fait que** les sels d'addition avec un acide des précurseurs de colorants d'oxydation et des coupleurs sont choisis parmi les chlorhydrates, les bromhydrates, les sulfates, les tartrates, les lactates et les acétates.

12. Composition selon l'une quelconque des revendications précédentes, **caractérisée par le fait qu**'elle contient en outre des colorants directs.

13. Composition selon l'une quelconque des revendications 1 et 6 **caractérisée par le fait qu**'elle contient en outre au moins un polymère substantif cationique ou amphotère.

14. Composition selon la revendication 13, **caractérisée par le fait que** le polymère est un polymère de polyammonium quaternaire constitué de motifs récurrents répondant à la formule (IV) suivante :

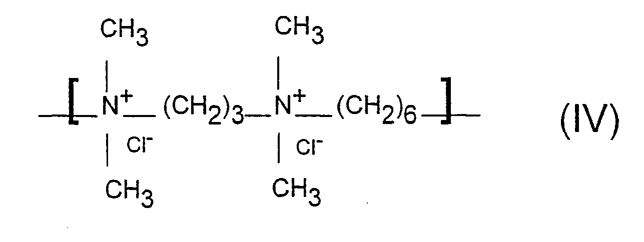

15. Composition selon la revendication 13, **caractérisée par le fait que** le polymère est un polymère de polyammonium quaternaire constitué de motifs récurrents répondant à la formule (V) suivante :

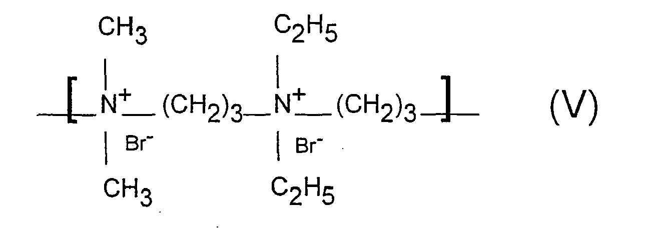

16. Composition selon l'une quelconque des revendications précédentes, **caractérisée par le fait qu**'elle contient en outre au moins un agent réducteur, présent dans des quantités allant de 0,05 à 1,5% en poids par rapport au poids total de la composition.

17. Composition selon l'une quelconque des revendications précédentes, **caractérisée par le fait qu**'elle contient en outre un agent oxydant.

18. Composition selon la revendication 17, **caractérisée par le fait qu**'elle possède un pH allant de 4 à 11.

19. Composition selon la revendication 17, **caractérisée par le fait que** l'agent oxydant est choisi parmi le peroxyde d'hydrogène, le peroxyde d'urée, les bromates et les ferricyanures de métaux alcalins, et les persels.

20. Composition selon les revendications 17 ou 19, **caractérisée par le fait que** l'agent oxydant est une solution d'eau oxygénée dont le titre varie de 2,5 à 40 volumes.

21. Composition selon l'une quelconque des revendications précédentes, **caractérisée par le fait que** les polymères amphiphiles non-ioniques comportant au moins une chaîne grasse et au moins un motif hydrophile sont utilisés en une quantité allant de 0,05 à 10% en poids du poids total de la composition appliquée sur les fibres et encore plus préférentiellement de 0,2 à 5%.

22. Procédé de teinture des fibres kératiniques et en particulier des fibres kératiniques humaines telles que les cheveux, **caractérisé par le fait qu**'il consiste à appliquer sur les fibres une composition de teinture (A1) telle que définie à l'une quelconque des revendications 1 à 16, et à révéler la couleur en milieu alcalin, neutre ou acide à l'aide d'un agent oxydant qui est ajouté juste au moment de l'emploi à cette composition (A1) ou qui est présent dans une composition (B1) appliquée séquentiellement sans rinçage intermédiaire.

23. Procédé de teinture des fibres kératiniques et en particulier des fibres kératiniques humaines telles que les cheveux, **caractérisé par le fait qu**'il consiste à appliquer sur les fibres une composition de teinture (A2) contenant dans un milieu approprié pour la teinture au moins un précurseur de colorant d'oxydation, éventuellement un ou plusieurs coupleurs, en la présence ou en l'absence d'un polymère amphiphile non-ionique comportant au moins une chaîne grasse et au moins un motif hydrophile tel que défini à l'une quelconque des revendications 1 à 6, et à révéler la couleur en milieu alcalin, neutre ou acide à l'aide d'une composition oxydante (B2) contenant un agent oxydant et une quantité efficace d'au moins un polymère amphiphile non-ionique comportant au moins une chaîne grasse et au moins un motif hydrophile tel que défini à l'une quelconque des revendications 1 à 6, et qui est mélangée juste au moment de l'emploi à la composition (A2) ou qui est appliquée séquentiellement sans rinçage intermédiaire.

24. Procédé de teinture selon la revendication 23, **caractérisé par le fait que** la composition (A2) et/ou la composition (B2) renferment au moins un polymère substantif cationique ou amphotère.

25. Dispositif à plusieurs compartiments ou « Kit » pour la teinture des fibres kératiniques et en particulier des fibres kératiniques humaines telles que les cheveux, **caractérisé par le fait qu**'il comporte au moins deux compartiments, dont l'un d'entre eux renferme une composition (A1) telle que définie à l'une quelconque des revendications 1 à 16, et un autre une composition (B1) comprenant un agent oxydant dans un milieu approprié pour la teinture.

26. Dispositif à plusieurs compartiments ou « Kit » pour la teinture des fibres kératiniques et en particulier des fibres kératiniques humaines telles que les cheveux, **caractérisé par le fait qu**'il comporte au moins deux compartiments, dont l'un d'entre eux renferme une composition (A2) telle que définie dans les revendications 23 et 24, et un autre une composition (B2) telle que définie dans les revendications 23 et 24.

27. Utilisation d'une composition de teinture d'oxydation telle que définie à l'une quelconque des revendications 1 à 21 ou d'un dispositif de teinture ou « Kit » à plusieurs compartiments tel que défini à la revendication 25 ou 26, pour la teinture d'oxydation des fibres kératiniques humaines telles que les cheveux.

## Claims

1. Composition for the oxidation dyeing of keratin fibres, in particular human keratin fibres such as the hair, of the type comprising, in a medium which is suitable for dyeing, at least one oxidation dye precursor and, where appropriate, one or more couplers, **characterized in that** it also contains at least one nonionic amphiphilic polymer containing at least one fatty chain and at least one hydrophilic unit chosen from the group consisting of nonionic celluloses modified with groups containing at least one fatty chain, hydroxypropyl guars modified with groups containing at least one fatty chain, polyether urethanes containing at least one fatty chain;
with the proviso that:
(1) the said composition is different from a composition containing Aculyn 44 (INCI name: PEG 150/Decyl Alcohol/SMDI Copolymer), an oxidizing preformed organic peroxy acid and one or more hair oxidation dyes;
(2) the said composition is different from a composition containing Aculyn 44 (INCI name: PEG 150/Decyl Alcohol/SMDI Copolymer), an oxidizing water-soluble peroxygen compound, an oxidizing organic peroxy acid precursor and one or more hair oxidation dyes;
(3) the said composition is different from a composition containing 5% hydrogen peroxide, 0.8% para-phenylenediamine, 0.2% para-aminophenol, 1% meta-aminophenol, 1.6% resorcinol, 1% nonoxynol-9, 0.1% ethylenediaminetetraacetic acid, 0.5% Aculyn 44 (INCI name: PEG 150/Decyl Alcohol/SMDI Copolymer) and water.

2. Composition according to Claim 1, **characterized in that** the celluloses are hydroxyethyl-celluloses modified with groups containing at least one alkyl, arylalkyl or alkylaryl group.

3. Composition according to Claim 2, **characterized in that** the cellulose is a hydroxyethyl cellulose modified with groups containing at least one C₁₆ alkyl group.

4. Composition according to Claim 1, **characterized in that** the celluloses are hydroxyethyl-celluloses modified with groups containing at least one polyalkylene glycol alkylphenyl ether group.

5. Composition according to Claim 4, **characterized in that** the cellulose is a hydroxyethylcellulose modified with groups containing at least one polyethylene glycol (15) nonylphenyl ether group.

6. Composition according to Claim 1, **characterized in that** the polyether urethanes are modified with at least one C₈-C₃₀ alkyl or alkenyl group.

7. Composition according to any one of the preceding claims, **characterized in that** the oxidation dye precursors are chosen from ortho- or para- phenylenediamines, bis(phenyl)alkylenediamines, ortho- or para-aminophenols and heterocyclic bases, and the addition salts thereof with an acid.

8. Composition according to any one of the preceding claims, **characterized in that** the oxidation dye precursors are present in concentrations ranging from 0.0005 to 12% by weight relative to the total weight of the composition.

9. Composition according to any one of the preceding claims, **characterized in that** the couplers are chosen from meta-phenylenediamines, meta-aminophenols, meta-diphenols and heterocyclic couplers, and the addition salts thereof with an acid.

10. Composition according to any one of the preceding claims, **characterized in that** the couplers are present in concentrations ranging from 0.0001 to 10% by weight relative to the total weight of the composition.

11. Composition according to any one of the preceding claims, **characterized in that** the addition salts with an acid of the oxidation dye precursors and of the couplers are chosen from the hydrochlorides, hydrobromides, sulphates, tartrates, lactates and acetates.

12. Composition according to any one of the preceding claims, **characterized in that** it also contains direct dyes.

13. Composition according to either of Claims 1 and 6, **characterized in that** it also contains at least one cationic or amphoteric substantive polymer.

14. Composition according to Claim 13, **characterized in that** the polymer is a quaternary polyammonium polymer consisting of repeating units corresponding to formula (IV) below:

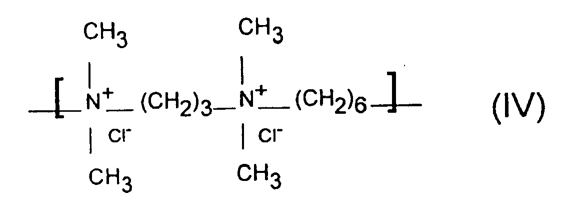

15. Composition according to Claim 13, **characterized in that** the polymer is a quaternary polyammonium polymer consisting of repeating units corresponding to formula (V) below:

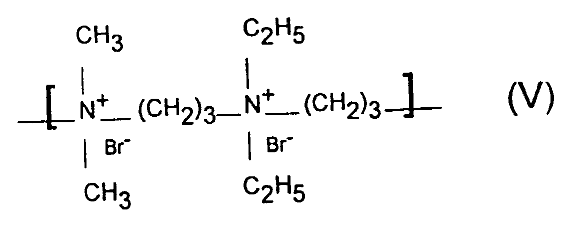

16. Composition according to any one of the preceding claims, **characterized in that** it also contains at least one reducing agent which is present in amounts ranging from 0.05 to 1.5% by weight relative to the total weight of the composition.

17. Ready-to-use composition according to any one of the preceding claims, **characterized in that** it also contains an oxidizing agent.

18. Composition according to Claim 17, **characterized in that** it has a pH ranging from 4 to 11.

19. Composition according to Claim 17, **characterized in that** the oxidizing agent is chosen from hydrogen peroxide, urea peroxide, alkali metal bromates and ferricyanides, and persalts.

20. Composition according to Claims 17 or 19, **characterized in that** the oxidizing agent is an aqueous hydrogen peroxide solution whose titre ranges from 2.5 to 40 volumes.

21. Composition according to any one of the preceding claims, **characterized in that** the nonionic amphiphilic polymers containing at least one fatty chain and at least one hydrophilic unit are used in an amount ranging from 0.05 to 10% by weight relative to the total weight of the composition applied to the fibres, and even more preferably from 0.2 to 5%.

22. Process for dyeing keratin fibres, and in particular human keratin fibres such as the hair, **characterized in that** it consists in applying to the fibres a dye composition (A1) as defined in any one of Claims 1 to 16, and in developing the colour in alkaline, neutral or acidic medium using an oxidizing agent which is added to this composition (A1) only at the time of use or which is present in a composition (B1) that is applied sequentially without intermediate rinsing.

23. Process for dyeing keratin fibres, and in particular human keratin fibres such as the hair, **characterized in that** it consists in applying to the fibres a dye composition (A2) containing, in a medium which is suitable for dyeing, at least one oxidation dye precursor and optionally one or more couplers, in the presence or absence of a nonionic amphiphilic polymer containing at least one fatty chain and at least one hydrophilic unit as defined in any one of Claims 1 to 6, and in developing the colour in alkaline, neutral or acidic medium using an oxidizing composition (B2) which contains an oxidizing agent and an effective amount of at least one nonionic amphiphilic polymer containing at least one fatty chain and at least one hydrophilic unit as defined in any one of Claims 1 to 6, and which is mixed with the composition (A2) only at the time of use or which is applied sequentially without intermediate rinsing.

24. Dyeing process according to Claim 23, **characterized in that** the composition (A2) and/or the composition (B2) contain at least one cationic or amphoteric substantive polymer.

25. Multi-compartment kit or device for dyeing keratin fibres, and in particular human keratin fibres such as the hair, **characterized in that** it contains at least two compartments, one of which contains a composition (A1) as defined in any one of Claims 1 to 16, and another contains a composition (B1) comprising an oxidizing agent in a medium which is suitable for dyeing.

26. Multi-compartment kit or device for dyeing keratin fibres, and in particular human keratin fibres such as the hair, **characterized in that** it contains at least two compartments, one of which contains a composition (A2) as defined in Claims 23 and 24, and another contains a composition (B2) as defined in Claims 23 and 24.

27. Use of an oxidation dye composition as defined in any one of Claims 1 to 21 or of a multi-compartment kit or device for dyeing as defined in Claim 25 or 26, for the oxidation dyeing of human keratin fibres such as the hair.

## Patentansprüche

1. Zusammensetzung zum oxidativen Färben von Keratinfasern und insbesondere menschlichen Keratinfasern, wie Haaren, die in einem zum Färben geeigneten Medium mindestens ein Farbstoffvorprodukt eines Oxidationsfarbstoffes und gegebenenfalls einen oder mehrer Kuppler enthält, **dadurch gekennzeichnet, daß** sie ferner mindestens ein amphiphiles nichtionisches Polymer enthält, das mindestens eine Fettkette und mindestens eine hydrophile Gruppe aufweist und das unter den nichtionischen Cellulosen, die mit Gruppen modifiziert sind, die mindestens eine Fettkette aufweisen, Hydroxypropylguarverbindungen, die mit Gruppen modifiziert sind, die mindestens eine Fettkette aufweisen, und Urethanpolyethern, die mindestens eine Fettkette aufweisen, ausgewählt ist, mit der Maßgabe, dass:
(1) die Zusammensetzung von einer Zusammensetzung verschieden ist, die Acuyln 44 (INCI-Bezeichung: PEG-150/Decyl Alcohol/SMDI Copolymer), eine oxidierende, vorab gebildete organische Peroxysäure und einen oder mehrere Oxidationsfarbstoffe für das Haar enthält;
(2) die Zusammensetzung von einer Zusammensetzung verschieden ist, die Acuyln 44 (INCI-Name: PEG-150/Decyl Alcohol/ SMDI Copolymer), einen oxidierenden, wasserlöslichen Peroxybildner, einen Vorläufer einer oxidierenden, organischen Peroxysäure und einen oder mehrere Oxidationsfarbstoffe für das Haar enthält;
(3) die Zusammensetzung von einer Zusammensetzung verschieden ist, die 5 % Wasserstoffperoxid, 0,8 % p-Phenylendiamin, 0,2 % p-Aminophenol, 1 % m-Aminophenol, 1,6 % Resorcin, 1 % Nonoxynol-9, 0,1 % Ethylendiamintetraessigsäure, 0,5 % Acuyln 44 (INCI-Bezeichung: PEG-150/Decyl Alcohol/SMDI Copolymer) und Wasser enthält.

2. Zusammensetzung nach Anspruch 1, **dadurch gekennzeichnet, daß** die Cellulosen Hydroxyethylcellulosen sind, die mit Gruppen modifiziert sind, die mindestens eine Alkyl-, Arylalkyl- oder Alkylarylgruppe enthalten.

3. Zusammensetzung nach Anspruch 2, **dadurch gekennzeichnet, daß** die Cellulosen Hydroxyethylcellulosen sind, die mit Gruppen modifiziert sind, die mindestens eine C₁₆-Alkylgruppe enthalten.

4. Zusammensetzung nach Anspruch 1, **dadurch gekennzeichnet, daß** die Cellulosen Hydroxyethylcellulosen sind, die mit Gruppen modifiziert sind, die mindestens eine Polyalkylenglykolalkylphenolethergruppe enthalten.

5. Zusammensetzung nach Anspruch 4, **dadurch gekennzeichnet, daß** die Cellulosen Hydroxyethylcellulosen sind, die mit Gruppen modifiziert sind, die mindestens einen Polyethylenglykol(15)ether von Nonylphenol enthalten.

6. Zusammensetzung nach Anspruch 1, **dadurch gekennzeichnet, daß** die Urethanpolyether mit mindestens einer C₈₋₃₀-Alkyl- oder Alkenylgruppe modifiziert sind.

7. Zusammensetzung nach einem der vorhergehenden Ansprüche, **dadurch gekennzeichnet, daß** die Farbstoffvorprodukte von Oxidationsfarbstoffen unter den o- oder p-Phenylendiaminen, Bisphenylalkylendiaminen, o- oder p-Aminophenolen und heterocyclischen Basen sowie den Additionssalzen dieser Verbindungen mit einer Säure ausgewählt sind.

8. Zusammensetzung nach einem der vorhergehenden Ansprüche, **dadurch gekennzeichnet, daß** die Farbstoffvorprodukte von Oxidationsfarbstoffen in einem Mengenanteil von 0,0005 bis 12 Gew.-%, bezogen auf das Gesamtgewicht der Zusammensetzung, vorliegen.

9. Zusammensetzung nach einem der vorhergehenden Ansprüche, **dadurch gekennzeichnet, daß** die Kuppler unter den m-Phenylendiaminen, m-Aminophenolen, m-Dihydroxybenzolen und heterocyclischen Kupplern und den Additionssalzen dieser Verbindungen mit einer Säure ausgewählt sind.

10. Zusammensetzung nach einem der vorhergehenden Ansprüche, **dadurch gekennzeichnet, daß** die Kuppler in einem Mengenanteil von 0,0001 bis 10 Gew.-%, bezogen auf das Gesamtgewicht der Zusammensetzung, vorliegen.

11. Zusammensetzung nach einem der vorhergehenden Ansprüche, **dadurch gekennzeichnet, daß** die Additionssalze der Farbstoffvorprodukte von Oxidationsfarbstoffen und der Kuppler mit einer Säure unter den Hydrochloriden, Hydrobromiden, Sulfaten, Tartraten, Lactaten und Acetaten ausgewählt sind.

12. Zusammensetzung nach einem der vorhergehenden Ansprüche, **dadurch gekennzeichnet, daß** sie ferner Direktfarbstoffe enthält.

13. Zusammensetzung nach den Ansprüchen 1 und 6, **dadurch gekennzeichnet, daß** sie ferner mindestens ein kationisches oder amphoteres substantives Polymer enthält.

14. Zusammensetzung nach Anspruch 13, **dadurch gekennzeichnet, daß** das Polymer ein quartäres Polyammoniumpolymer ist, das aus wiederkehrenden Einheiten der folgenden Formel (IV) besteht:

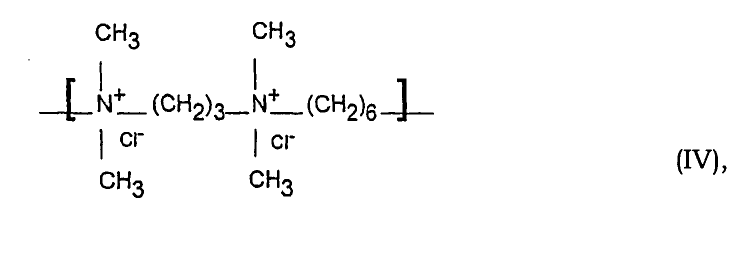

15. Zusammensetzung nach Anspruch 13, **dadurch gekennzeichnet, daß** das Polymer ein quartäres Polyammoniumpolymer ist, das aus wiederkehrenden Einheiten der folgenden Formel (V) besteht:

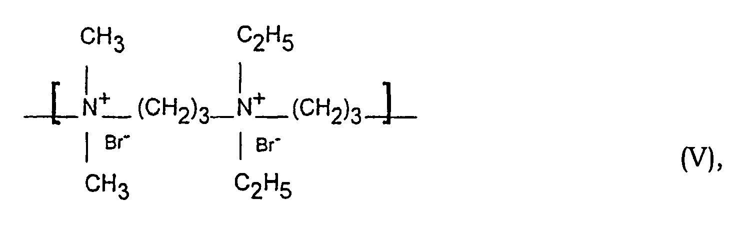

16. Zusammensetzung nach einem der vorhergehenden Ansprüche, **dadurch gekennzeichnet, daß** sie ferner mindestens ein Reduktionsmittel in einer Menge von 0,05 bis 1,5 Gew.-%, bezogen auf das Gesamtgewicht der Zusammensetzung, enthält.

17. Zusammensetzung nach einem der vorhergehenden Ansprüche, **dadurch gekennzeichnet, daß** sie ferner ein Oxidationsmittel enthält.

18. Zusammensetzung nach Anspruch 17, **dadurch gekennzeichnet, daß** sie einen pH-Wert von 4 bis 11 aufweist.

19. Zusammensetzung nach Anspruch 17, **dadurch gekennzeichnet, daß** das Oxidationsmittel unter Wasserstoffperoxid, Harnstoffperoxid, Bromaten und Ferricyaniden von Alkalimetallen und Salzen von Persäuren ausgewählt ist.

20. Zusammensetzung nach Anspruch 17 oder 19, **dadurch gekennzeichnet, daß** das Oxidationsmittel eine Wasserstoffperoxidlösung mit einem Titer von 2,5 bis 40 Volumina ist.

21. Zusammensetzung nach einem der vorhergehenden Ansprüche, **dadurch gekennzeichnet, daß** die nichtionischen amphiphilen Polymere, die mindestens eine Fettkette und mindestens eine hydrophile Gruppe aufweisen, in einem Mengenanteil von 0,05 bis 10 Gew.-% und vorzugsweise 0,2 bis 5 Gew.-%, bezogen auf das Gesamtgewicht der auf die Fasern aufgebrachten Zusammensetzung, verwendet werden.

22. Verfahren zum Färben von Keratinfasern und insbesondere menschlichen Keratinfasern, wie dem Haar, **dadurch gekennzeichnet, daß** es darin besteht, auf die Fasern eine Zusammensetzung (A1) nach einem der Ansprüche 1 bis 16 aufzutragen, und die Farbe in einem alkalischen, neutralen oder sauren Medium mit einem Oxidationsmittel zu entwickeln, das bei der Anwendung zu der Zusammensetzung (A1) gegeben wird oder das in einer oxidierenden Zusammensetzung (B1) vorliegt, die getrennt davon anschließend ohne zwischenzeitliches Spülen aufgebracht wird.

23. Verfahren zum Färben von Keratinfasern und insbesondere menschlichen Keratinfasern, wie dem Haar, **dadurch gekennzeichnet, daß** es darin besteht, auf die Fasern eine Farbmittelzusammensetzung (A2) aufzutragen, die in einem zum Färben geeigneten Medium mindestens ein Farbstoffvorprodukt eines Oxidationsfarbstoffes und gegebenenfalls einen oder mehrere Kuppler und gegebenenfalls ein amphiphiles nichtionisches Polymer, das mindestens eine Fettkette und mindestens eine hydrophile Gruppe aufweist, nach einem der Ansprüche 1 bis 6 enthält, und die Farbe in einem alkalischen, neutralen oder sauren Medium mit einer oxidierenden Zusammensetzung (B2) zu entwickeln, die ein Oxidationsmittel und in einer wirksamen Menge mindestens ein amphiphiles nichtionisches Polymer, das mindestens eine Fettkette und mindestens eine hydrophile Gruppe aufweist, nach einem der Ansprüche 1 bis 6 enthält und die bei der Anwendung mit der Zusammensetzung (A2) vermischt wird oder getrennt davon anschließend ohne zwischenzeitliches Spülen aufgebracht wird.

24. Verfahren zum Färben nach Anspruch 23, **dadurch gekennzeichnet, daß** die Zusammensetzung (A2) und/oder die Zusammensetzung (B2) mindestens ein kationisches oder amphoteres substantives Polymer enthält.

25. Vorrichtung mit mehreren Abteilungen oder "Kit" zum Färben von Keratinfasern und insbesondere menschlichen Keratinfasern, wie dem Haar, **dadurch gekennzeichnet, daß** sie mindestens zwei Abteilungen aufweist, wobei eine Abteilung eine Zusammensetzung (A1) nach einem der Ansprüche 1 bis 16 und eine weitere Abteilung eine Zusammensetzung (B1) enthält, die in einem zum Färben geeigneten Medium ein Oxidationsmittel enthält.

26. Vorrichtung mit mehreren Abteilungen oder "Kit" zum Färben von Keratinfasern und insbesondere menschlichen Keratinfasern, wie dem Haar, **dadurch gekennzeichnet, daß** sie mindestens zwei Abteilungen aufweist, wobei eine Abteilung mindestens eine Zusammensetzung (A2) nach einem der Ansprüche 23 und 24 und eine weitere Abteilung eine Zusammensetzung (B2) nach einem der Ansprüche 23 und 24 enthält.

27. Verwendung einer Zusammensetzung zum oxidativen Färben nach einem der Ansprüche 1 bis 21 oder einer Vorrichtung zum Färben oder "Kit" mit mehreren Abteilungen nach einem der Ansprüche 25 oder 26 zum oxidativen Färben von menschlichen Keratinfasern, wie dem Haar.
